# EUROPEAN PATENT APPLICATION

(11) **EP 4 488 384 A1**
(43) Date of publication of application: **08.01.2025**
(21) Application number: 23183637.0
(22) Date of filing: 05.07.2023
(51) Int. Cl.: C12Q 1/6813, C12Q 1/6827, C12Q 1/6844, C12Q 1/6853, C12Q 1/6858

(54) **PRIMERS FOR SELECTIVE AMPLIFICATION OF RARE TARGET SEQUENCES**

(71) Applicant: Infiniplex Ltd., 2069205 Yokneam Illit (IL)
(72) Inventor: Dr. Peleg, Ofer, 5290595 Ramat Gan (IL)
(74) Representative: Fabry, Bernd

(57) **Abstract**

The present invention discloses multi-part amplification primers that distinguish between a mutant DNA target sequence and a closely related wild-type DNA target sequence. Further disclosed are methods for the detection of low copy targets in qPCR reactions using discloses primers

## Description

### FIELD OF THE INVENTION

The present invention relates to nucleic acid amplification primers useful for the selective amplification and/or detection of mutant DNA target sequences with exceptional low copy number comprising samples such as liquid biopsies with excess wildtype target DNA sequences for diagnostic purposes. Said primers are particularly useful for the specific and selective amplification and/or detection of rare target sequences differing from highly abundant sequences in as little as one nucleotide. Further disclosed are methods for the amplification and/or detection of rare nucleic acid targets in qPCR reactions using said primers as well as primer designs and kits.

### BACKGROUND OF THE INVENTION

Confident detection and quantification of target nucleic acid sequences in samples comprising low concentrations of the target may require the selective amplification of said target using Polymerase Chain Reaction (PCR) and real-time detection (RT) of the generated amplification products (amplicons) in a method known as RT-qPCR. The selection of specific primers comprising short DNA sequences complementary to the target sequences is critical to ensure the specific amplification of the target, while preventing unspecific amplification of closely related sequences present in the sample. Accordingly, primer sequences are designed to be complementary to the target in order to allow hybridization of the primers to the target DNA sequence and its amplification products. The selection of a specific annealing temperatures may prevent the annealing of the specific primer to closely related target sequences by favoring annealing to the (near-) perfectly complementary target sequence. However, specific amplification of highly similar sequences differing in as little as one or two nucleotides can be challenging using conventional DNA primers.

This is particularly challenging when distinguishing between mutant and wild-type sequences differing only by single-nucleotide polymorphisms (SNPs). While designing amplification primers with complete complementary to a mutant DNA target sequences can favor the amplification of the corresponding target, wild-type sequences may be also amplified due to highly similar properties such as annealing temperatures and extensive base complementarity. The undesired amplification of the wild-type DNA target sequence may be less efficient compared to the intended mutant DNA target sequence. However, detection and quantification of mutant and wild-type target sequences becomes unreliable.

This is particularly problematic in diagnostics, where the detection of mutant DNA target sequences in samples comprising predominantly wild-type DNA target sequences is critical. The presence of the wild-type DNA sequence masks the low copy numbers of the mutant DNA target sequence due to insufficient selectivity of amplification caused by the conventional primer design.

One example of these challenges posed by the low selectivity of conventional primer designs is the identification of mutant DNA target sequences in circulating cell-free DNA. Circulating cell-free DNA (cfDNA) is recognized to have reasonable prognostic and diagnostic potential in several pathological diseases including cancer, sepsis, and autoimmune diseases such as systemic lupus erythematosus (SLE). Cancer is one of the deadliest diseases and projected to be responsible for a significant proportion of deaths in our aging society. The detection of cancers in early stages is associated with improved outlook on health and survival rates. While cancer symptoms can arise at late stages of the disease, multiple approaches are available to detect cancers before symptoms are present. For instance, extensive screenings are common at certain ages in order to identify cancerous lesions early and start treatment. However, current diagnostics fail short in providing a cost-effective and rapid identification of early stages of cancer.

Cancer patients usually have a high level of cfDNA in their serum or plasma as a result of cellular necrosis or apoptosis, since tumor cells divide faster than normal cells, and cfDNAs are released in a high proportion (Sorenson et al., 1994; Vasioukhin et al., 1994; Raja et al., 2018). The fraction of cfDNA derived from tumor cells is named circulating tumor DNA (ctDNA) (Leon et al., 1977; Shu et al., 2017). In recent years, both cfDNA and ctDNA have received increased attention as novel blood biomarkers, as quantification and kinetic analysis of cfDNA (Diehl et al., 2008), and molecular profiling of ctDNA have suggested their predictive and prognostic values (lizuka et al., 2006; Tokuhisa et al., 2007). Several liquid biopsy tests, designed for the identification of cancer-specific mutations, have been recommended as companion diagnostic (CDx) tests, by the European Medicines Agency (EMA) and Food and Drug Administration (FDA) of USA, to guide therapeutic decision making.

However, the identification of mutant DNA target sequences such as ctDNA requires the specific detection of the mutated sequence and selective distinguishing between the mutant and wild-type target. Most recently, the isolation of cfDNA and subsequent sequencing using next-generation sequencing (NGS) approaches has been a powerful tool to not only detect the targeted sequence but also distinguish between mutant and wild-type sequence. However, the low copy number of the mutant compared to the wild-type sequence requires extensive sample preparations and deep sequencing of samples significantly increasing cost and time commitment. Accordingly, NGS approaches are currently insufficient to provide rapid, frequent, and cost-effective diagnostic screenings.

Accordingly, there is a significant need for novel methods able to rapidly screen samples for mutant DNA target sequences, while keeping costs low. qPCR detection is a well-established method for the identification and quantification of nucleic acid targets in clinical samples. However, conventional primers fall short in providing amplification efficiency and selectivity to reliably identify mutant DNA sequences at very low concentrations differing by as little as one or two bases from the wild-type allele. Innovation in primer design approaches have significantly increased efficiency of amplification. For instance, the incorporation of ribonucleotides at selected positions in conventional primer design has decreased primer dimer formation thus highly increasing PCR efficiency (e.g., WO2009004630A1).

Additionally, the development of highly selective amplification primers addresses some needs for the ability to specifically and selectively amplify a mutant DNA target sequence over a closely related wild-type sequence. Multiple approaches aiming to increase selectivity of primers have been disclosed such as hairpin-shaped primers (e.g., WO 2000/71562), Amplification Refractory Mutation System (ARMS) primers (Curr Protoc Hum Genet. 2001 May;Chapter 9:Unit 9.8. doi: 10.1002/0471142905.hg0908s07.) and multi-part primers such as SuperSelective primers (WO 2014/124290, WO2017176852A1, WO2021067527A1) containing an internal sequence that is not complementary to the target sequence in between two target-complementary sequences.

While significant progress has been made, selectivity during PCR amplification remains challenging. Accordingly, there is a significant need for improved and/or alternative primers and amplification methods allowing the selective amplification of a mutant DNA target sequence, wherein a closely related wild-type DNA target sequences is not amplified or at very low levels allowing cost-effective, rapid, and non-invasive diagnostics.

The objective problem of the invention was, therefore, to develop novel primers and primer designs that enable the specific and selective amplification of mutant DNA target sequences over closely related wild-type sequences, useful in quantitative assays in order to increase detection sensitivity and reliability of such approaches and allow for cost-effective and conclusive screening procedures.

### SUMMARY OF THE INVENTION

A first aspect of the present invention relates to a multi-part amplification primer that distinguishes between a mutant DNA target sequence and a closely related wild-type DNA target sequence characterized in that it consists of or comprises, in the 5' to 3' direction, the following five contiguous DNA sequences,
(i) a docking sequence, capable of forming a hybrid 15-40 nucleotides in length with the mutant DNA target sequence and with the wildtype DNA target sequence,
(ii) a hairpin sequence incapable of forming a hybrid with the mutant DNA target sequence and with the wildtype DNA target sequence,
(iii) a spacer sequence,
(iv) a palm sequence complementary to the mutant DNA target sequence and wildtype DNA target sequence capable of forming a hybrid 4 to 10 nucleotides in length with the hairpin sequence and
(v) a mutation-recognizing sequence, comprising a mutation-specific nucleotide at the first or second 5'position of the sequence,
wherein
the contiguous hairpin sequence, spacer sequence, and palm sequences form a stem-loop structure,
the mutant target DNA sequence is perfectly complementary to the contiguous sequence formed by the palm sequence and the mutation-recognizing sequence, and the wild-type DNA target sequence is mismatched to said contiguous sequence with the mutation-specific nucleotide of the mutation-recognizing sequence.

In some embodiments, said docking sequence further comprises at the ultimate 3'position a dimer-preventing nucleotide that does not pair with the mutation-specific nucleotide and/or with any other nucleotide at the 3' end position of the switch primer.. In some embodiments, said dimer-preventing nucleotide is the same nucleotide as the first 5' nucleotide of the mutation-recognizing sequence. In some embodiments, said dimer-preventing nucleotide is a locked nucleic acid (LNA).

In some embodiments, said multi-part amplification primer further comprises at least one ribonucleotide. In a preferred embodiment, said the at least one ribonucleotide is placed within the last 10 nucleotides of the 3' terminus of the primer, no two ribonucleotides are adjacent to one another, and the 3' terminal base is a deoxyribonucleotide.

A further aspect of the present invention relates to a method utilizing said multi-part amplification primers of the invention. Accordingly, further disclosed is a primer-dependent amplification and detection method that is capable of amplifying and detecting in a sample at least one mutant DNA target sequence in a mixture containing, for each mutant DNA target sequence, a closely related wild-type DNA target sequence that differs from the mutant DNA target sequence by as little as one or two base pairs, comprising consisting or essentially consisting of the following steps:
(a) providing a sample comprising the at least one mutant and/or wild-type DNA target sequence, and for each mutant DNA target sequence a pair of a forward primer and a reverse primer,
(b) preparing a primer-dependent amplification reaction mixture comprising or consisting of a DNA polymerase, deoxyribonucleoside triphosphates, an amplification buffer, other reagents required for amplification, the sample and the pair of the forward primer and reverse primer for each mutant DNA target sequence of step (a),
(c) repeatedly cycling the mixture thus obtained in a primer-dependent amplification reaction, under primer annealing conditions, having a primer-annealing temperature, to amplify each mutant DNA target sequence present in the sample, and
(d) detecting said at least one mutant DNA target sequence by measuring the quantity of the amplification products thus obtained;
wherein
the forward and/or reverse primer of the primer pair for each mutant DNA target sequence is a multi-part amplification primer according to any of the preceding claims characterized in that it is specific for the mutated DNA target sequence but mismatched to the wild-type DNA target sequence,
the docking sequence hybridizes at the primer-annealing temperature with the mutant DNA target sequence and with its closely related wild-type DNA target sequence.

In some embodiments, the method according to the invention is capable of amplifying and detecting in a sample as few as 10 copies of at least one mutant DNA target sequence in a mixture containing, for each mutant DNA target sequence, 10,000 copies of a closely related wild-type DNA target sequence that differs from the mutant DNA target sequence by as little as one or two base pairs.

In some embodiments of the method, either the forward or reverse primer of the primer pair for each mutant DNA target sequence is a conventional primer. In some embodiments of the method, the docking sequence and the palm sequence of the primer are hybridized either to the mutant DNA target sequence or to the wild-type DNA target sequence, and the probability that during said cycling a multi-part amplification primer/wild-type DNA target sequence hybrid will be extended is at least 1,000 times lower than the probability that during said cycling a multi-part amplification primer/mutant DNA target sequence hybrid will be extended as evidenced by a difference in cycle numbers (ΔCq) of at least ten thermal cycles.

In some embodiments of the method, the forward and reverse primers of said pair of forward primer and reverse primer are multi-part amplification primers according to the invention, the forward primer has a mutation-specific nucleotide that is complementary to the mutation site in one of the two strands of the mutant DNA target sequence, the reverse primer has a mutation-specific nucleotide that is complementary to the other mutant DNA target sequence of that base pair in the other of the two strands of the mutant DNA target sequence, whereby one primer binds to a target strand, and the other primer binds to the complementary target strand.

In some embodiments of the method, said closely related wild-type DNA target sequence differs from the mutant DNA target sequence by one base. In some embodiments, the docking sequence hybridizes with the mutant and wild-type DNA target sequence at the primer annealing temperature.

In some embodiments of the method, the primer-dependent amplification mixture of step (b) further comprises homogeneous fluorescence detection means for detecting amplification products, and step (d) comprises detecting said at least one mutant DNA target sequence by measuring the intensity of fluorescence from said homogeneous fluorescence detection means. In some embodiments, the primer-dependent amplification and detection method is a polymerase chain reaction (PCR), and detection is real-time (RT) detection.

In some embodiment of the method, said sample comprises cfDNA prepared from blood serum and/or plasma of a subject.

A further aspect relates to kit of reagents for performing the method of the invention. In some embodiments, said kit comprises, consists, or essentially consists of at least one pair of a forward primer and a reverse primer for one or more mutant DNA target sequence, dNTPs, a primer-dependent polymerase, a detection probe for each mutant DNA target and other reagents, particularly amplification buffer, needed for amplification

### DEFINITIONS

For convenience, certain terms employed in the specification, examples, and appended claims are collected here.

As used herein, the singular form "a," "an," and "the" include plural references unless the context clearly dictates otherwise. For example, the term "sample" also includes a plurality of samples.

A "conventional" primer is a single-stranded oligonucleotide that is 15-40 nucleotides in length, more usually 20-30 nucleotides in length, and that is perfectly complementary to the intended target. Any of several computer programs are commonly used to design conventional PCR primers.

As used herein the term "amplification" and its variants includes any process for producing multiple copies or complements of at least some portion of a polynucleotide, said polynucleotide typically being referred to as a "template." The template polynucleotide can be single stranded or double stranded. Amplification of a given template can result in the generation of a population of polynucleotide amplification products, collectively referred to as an "amplicon." The polynucleotides of the amplicon can be single stranded or double stranded, or a mixture of both. Typically, the template will include a target sequence, and the resulting amplicon will include polynucleotides having a sequence that is either substantially identical or substantially complementary to the target sequence. In some aspects, the polynucleotides of a particular amplicon are substantially identical, or substantially complementary, to each other; alternatively, in some aspects the polynucleotides within a given amplicon can have nucleotide sequences that vary from each other. Amplification can proceed in linear or exponential fashion, and can involve repeated and consecutive replications of a given template to form two or more amplification products. Some typical amplification reactions involve successive and repeated cycles of template-based nucleic acid synthesis, resulting in the formation of a plurality of daughter polynucleotides containing at least some portion of the nucleotide sequence of the template and sharing at least some degree of nucleotide sequence identity (or complementarity) with the template. In some aspects, each instance of nucleic acid synthesis, which can be referred to as a "cycle" of amplification, includes creating free 3' end (e.g., by nicking one strand of a dsDNA) thereby generating a primer and primer extension steps; optionally, an additional denaturation step can also be included wherein the template is partially or completely denatured. In some aspects, one round of amplification includes a given number of repetitions of a single cycle of amplification. For example, a round of amplification can include 5, 10, 15, 20, 25, 30, 35, 40, 50, or more repetitions of a particular cycle. In one exemplary aspect, amplification includes any reaction wherein a particular polynucleotide template is subjected to two consecutive cycles of nucleic acid synthesis. The synthesis can include template-dependent nucleic acid synthesis.

The term "primer" refers to a strand of nucleic acid or an oligonucleotide capable of hybridizing to a template nucleic acid and acting as the initiation point for incorporating extension nucleotides according to the composition of the template nucleic acid for nucleic acid synthesis. "Extension nucleotides" refer to any nucleotide capable of being incorporated into an extension product during amplification, i.e., DNA, RNA, or a derivative if DNA or RNA, which may include a label.

"Hybridization" or "hybridize" or "anneal" refers to the ability of completely or partially complementary nucleic acid strands to come together under specified hybridization conditions (e.g., annealing conditions) in a parallel or preferably antiparallel orientation to form a stable double-stranded structure or region (sometimes called a "hybrid") in which the two constituent strands are joined by hydrogen bonds. Although hydrogen bonds typically form between adenine and thymine or uracil (A and T or U) or cytosine and guanine (C and G), other base pairs may form (e.g., Adams et al., The Biochemistry of the Nucleic Acids, 11th ed., 1992).

As used herein, two nucleic acid sequences "complement" one another or are "complementary" to one another if they base pair one another at each position. The term "complement" is defined as a sequence which pairs to a given sequence based upon the canonic base-pairing rules. For example, a sequence A-G-T in one nucleotide strand is "complementary" to T-C-A in the other strand The term "complement" and the phrase "reverse complement" are used interchangeably herein with respect to nucleic acids, and are meant to define the antisense nucleic acid.

The terms "polynucleotide" and "nucleic acid" are used herein interchangeably. They refer to a polymeric form of nucleotides of any length: Polynucleotides may have any three-dimensional structure, and may perform any function, known or unknown. The following are non-limiting examples of polynucleotides: coding or non-coding regions of a gene or gene fragment, exons, introns, messenger RNA (mRNA), transfer RNA, ribosomal RNA, ribozymes, cDNA, synthetic polynucleotides, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified, such as by conjugation with a labeling component.

If not stated otherwise, the term "nucleic acid" refers to any nucleic acid such as ribonucleic acid, deoxyribonucleic acid, xeno nucleic acid, single stranded or double stranded.

The term "mutation site " refers to a sequence of the mutant and wild-type DNA target sequence that distinguishes the mutant from the wild-type. The mutation site may consist of a single mismatched nucleotide (such as a SNP) or multiple mismatched nucleotides.

Unless defined otherwise, the technical and scientific terms used herein have the same meaning as commonly understood by one of ordinary skill in the art to which the invention belongs. Particularly, unless otherwise stated, a term as used herein is given the definition as provided in the Oxford dictionary of biochemistry and molecular biology, Oxford University Press, 1997, revised 2000 and reprinted 2003, ISBN 0 19 850673 2.

### DETAILED DESCRIPTION OF THE INVENTION

The present invention refers to multi-part amplification primers for primer-dependent amplification reactions capable of distinguishing between a mutant DNA target sequence and a closely related wild-type DNA target sequence, which we call Switch primers. Switch primers show significantly increased selective amplification of desired mutant DNA target sequences and impeded amplification of a wild-type DNA target sequence compared to conventional primer designs or other approaches known in the art when hybridized to a mutant DNA target and the closely related sequence.

Following amplification of the desired mutant DNA target sequence comprising a sample such as liquid biopsy of a subject, amplicons are exponentially amplified with high efficiency, which allows the real-time detection of amplicon abundance and the identification of the presence of the mutant DNA target sequence in said sample. Accordingly, the present invention discloses the design and properties as well as methods for the use of the Switch primers.

As disclosed below in the description and examples, the Switch primers are sufficiently selective to amplify desired mutant DNA target sequences while suppressing the amplification of a wild-type DNA target sequence even when mutant and wild-type DNA target sequence are distinguished by a single nucleotide polymorphism (SNP). Surprisingly, Switch primers allow the identification of mutant DNA target sequences in a sample containing as little as 10 copies of the mutant DNA target sequence for every 10,000 wild-type DNA target sequences.

The multi-part amplification primer (Switch primer) according to the invention is characterized in that it consists of or comprises, in the 5' to 3' direction, the following five contiguous DNA sequences (i) to (v):
(i) a docking sequence, capable of forming a hybrid 15-40 nucleotides in length with the mutant DNA target sequence and with the wildtype DNA target sequence,
(ii) a hairpin sequence,
(iii) a spacer sequence,
(iv) a palm sequence complementary to the mutant DNA target sequence and wildtype DNA target sequence and
(v) a mutation-recognizing sequence, comprising a mutation-specific nucleotide at the first or second 5' position of the sequence.

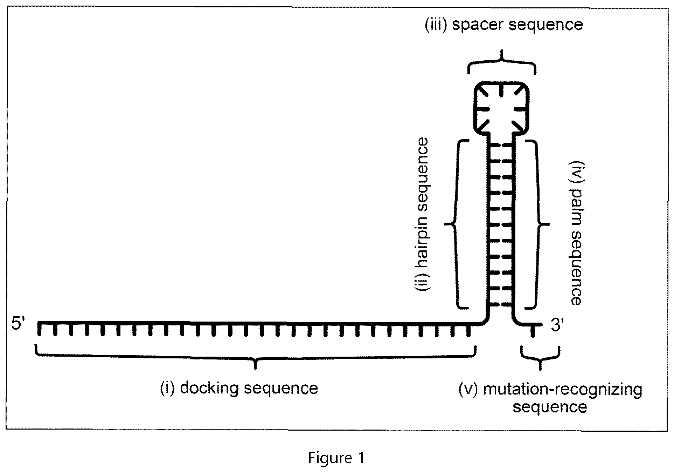

A schematic representation of an embodiment of the primer according to the invention can be found in Figure 1.

### TARGET SEQUENCES

There is a significant need by the industry to detect the presence and/or quantity of closely related sequences such as DNA sequences present in a sample. For instance, distinguishing between a mutant target DNA sequence and a wild-type DNA target sequence, such as an allele-specific mutation predictive of cancer and the wild-type allele, in samples such as liquid biopsies allow the detection of disease and subsequent targeted treatment. Accordingly, the mutant DNA target sequence according to the invention refers to a sequence comprising a polynucleotide contained in a sample in need of detection of the presence and/or quantification of said sequence.

In some embodiments, the mutant and wild-type DNA target sequences are double stranded DNA (dsDNA). In some embodiments the mutant and wild-type DNA target sequences are single stranded DNA (ssDNA).

### MUTATION SITE

A mutant DNA target sequence according to the invention may differ from a wild-type target sequence, which may be simultaneously contained in the sample, by a distinct mutation site. The mutation site refers to the mismatched base(s) of the mutant and wild-type target DNA sequences and their position within said sequences. In some embodiments the mutation site comprises the DNA target sequence and is one, two, three, or four mismatched nucleotides. In a preferred embodiment, the mutation site is two mismatched bases, more preferred a single mismatched base (nucleotide polymorphism (SNP)).

### DOCKING SEQUENCE (i)

The docking sequence (i) comprising the Switch primer according to the invention is capable to hybridize with a shared sequence of the mutant and wild-type DNA target sequence (docking target site) and allows the positioning of the primer in close spatial proximity of the mutation site of the mutant/wild-type DNA target sequence, which distinguishes the mutated from the wild-type sequence such as a single mismatched base (SNP). Hybridization may occur due to base complementarity of the whole or part of the docking sequence to the docking target site comprising the mutant and wild-type DNA target sequence.

Positioning of the Switch primer according to the present invention using the docking sequence in order to distinguish between mutant and wild-type DNA target sequence is critical for the ability of the primer to favor amplification of the mutant DNA target while reducing the chance of amplification of the wild-type counterpart sequence. The positioning of the docking sequence in close proximity to the mutation site allows a stable interaction of the docking sequence of the primer with its target prior to further engagements of the remaining complementary parts of the multi-part amplification primer according to the invention. In some embodiments, the docking sequence is partially complementary to the shared sequence of the mutant and wild-type DNA target sequence (docking target site). In a preferred embodiment, the docking sequence is completely complementary to the docking target site.

In some embodiments the docking sequence is about 200 to about 5 nucleotides in length, preferably about 100 to about 10 nucleotides, more preferably about 80 to about 10 nucleotides, more preferably about 70 to about 15 nucleotides, and most preferably about 50 to about 15 nucleotides.

In some embodiments, the docking sequence is capable of forming a hybrid about 10 to about 200 nucleotides in length, more preferred about 10 to about 100 nucleotides in length, more preferred about 15 to about 50 nucleotides in length, and most preferred about 15 to about 40 nucleotides in length with the mutant DNA target sequence and with the wildtype DNA target sequence.

In some embodiments the docking sequence hybridizes with the shared docking target site about 200 nucleotides to about 50 nucleotides, preferably about 100 to about 10 nucleotides, most preferably about 50 to about 10 nucleotides downstream of the mutation site of the targeted strand of the mutant and wild-type DNA target sequence, counted by the distance between the ultimate 5' hybridized nucleotide of the docking target site to the 3' first base of the mutation site on the target strand.

In some embodiments, the docking sequence comprises secondary structures. In some embodiments, the docking sequence comprises a stem-loop structure.

### HAIRPIN SEQUENCE (ii)

The 3' ultimate nucleotide of the docking sequence is linked to the first 5' nucleotide of the hairpin sequence (ii). The hairpin sequence may not share perfect complementary with the sequence directly upstream of the docking target site of the mutant and wild-type DNA sequence and may not hybridize with said sequences. The hairpin sequence (ii) forms a hybrid with the palm sequence (iv). In some embodiments the hairpin sequence (ii) is perfectly complementary to the palm sequence (iv). In some embodiment, the hairpin sequence is incapable of forming a hybrid with the mutant DNA target sequence and with the wildtype DNA target sequence.

While the docking sequence stably places the Switch primer on the shared DNA sequence of the target (docking target site) according to the invention by hybridizing with the target sequence at certain annealing conditions such as at a specific annealing temperature, the hairpin sequence may be incapable of forming a hybrid with the target sequence. Accordingly, in some embodiments the hairpin sequence does not hybridize with the target DNA sequence and/or is not associated with said target DNA sequence by base pairing at the annealing temperature of the docking sequence.

In some embodiments, the hairpin sequence according to the invention is of identical length as the palm sequence. In another embodiment, the hairpin sequence is between about 1 to about 5 bases longer compared to the palm sequence. In another embodiment, the hairpin sequence is between about 1 to about 3 bases shorter compared to the palm sequence

In some embodiments the hairpin sequence is about 50 to about 3 nucleotides in length, preferably about 20 to about 3 nucleotides, more preferably about 10 to about 4 nucleotides, more preferably about 8 to about 4 nucleotides, and most preferably about 7 to about 5 nucleotides.

### SPACER SEQUENCE (iii)

The 3'ultimate nucleotide of the hairpin sequence is linked to the first 5' nucleotide of the spacer sequence (iii). Said spacer sequence serves as a spacer between the hairpin sequence and the palm sequence to allow the formation of a hairpin two-dimensional structure (stem-loop) formed by the hairpin sequence, spacer sequence, and palm sequence. In some embodiments, the spacer sequence does not share complementarity to either the hairpin sequence (ii) or palm sequence (iv). In some embodiments, the spacer sequence (iii) does not hybridize with the target DNA sequence at the annealing temperature of the docking sequence (i). In certain embodiments, the spacer sequence forms a hybrid with the target DNA sequence.

In some embodiments, the spacer sequence is about 20 to about 3 nucleotides in length, preferably about 10 to about 4 nucleotides, more preferably 8 to about 4 nucleotides, and most preferably 5 to about 3 nucleotides.

### PALM SEQUENCE (iv)

The 3'ultimate nucleotide of the spacer sequence is linked to the first 5' nucleotide of the palm sequence (iv). The palm sequence consists of a sequence complementary to the mutant and wild-type DNA target sequence (palm target site). In some embodiments the hairpin sequence is perfectly complementary to the palm target site directly downstream of the mutation site of the mutant and wild-type DNA target sequence on the target strand.

The palm sequence shares complementary to the hairpin sequence allowing the formation of a hybrid between the hairpin sequence and the palm sequence. In some embodiments, the hairpin and the palm sequence are perfectly complementary to each other. In some embodiments, the hairpin sequence is the reverse-complement sequence of the palm sequence. In some embodiments, the palm sequence is capable of forming a hybrid about 3 to about 30 nucleotides in length, more preferred about 4 to about 20 nucleotides in length, and most preferred about 4 to about 10 nucleotides in length with the hairpin sequence.

In some embodiments, the ΔG of the stem loop structure comprising the hairpin sequence, the spacer sequence, and the palm sequence is about 0 to about -15, preferably about 0 to about -10, more preferably about 0 to about -8, and most preferably about -2 to about -8.

While the hairpin sequence and the spacer sequence may not show complete complementary to the inter site of the target DNA sequence, partial homology between the spacer and/or parts of the hairpin sequence and the inter sequence is possible.

The target DNA sequence downstream of the mutation site and the hairpin sequence compete with the palm sequence due to complementarity. In some embodiments, the target DNA sequence downstream of the mutation site is identical with the hairpin sequence.

### MUTATION-RECOGNIZING SEQUENCE (v)

The 3' ultimate nucleotide of the palm sequence is linked to the first 5' nucleotide of the mutation-recognizing sequence (v). The mutation-recognizing sequence comprises or consists of one to four nucleotides, wherein at least one nucleotide (mutation-specific nucleotide/ interrogating nucleotide) is characterized in that it is complementary to the mutation site of the mutant DNA target sequence while mismatching with the respective nucleotide at the mutation site of the wild-type DNA target sequence. In a preferred embodiment, the mutation-recognizing sequence consists of two, more preferred of one nucleotide. In some embodiments, the mutation-specific nucleotide is the first or the second nucleotide of the mutation-recognizing sequence. In a preferred embodiment, the mutation-specific nucleotide is the first nucleotide of the mutation-recognizing sequence.

In order to prevent or reduce the risk of the incorporation of the mutation-recognizing sequence in the stem-loop formed by hairpin sequence, spacer sequence, and palm sequence, and allow optimal target engagement, the docking sequence may further comprise at the ultimate 3' position a dimer-preventing nucleotide that does not pair with the mutation-specific nucleotide and/or with any other nucleotide at the 3' end position of the switch primer.. In some embodiment, the dimer-preventing nucleotide is a locked nucleic acid (LNA). In some embodiments, the dimer-preventing nucleotide is a ribonucleotide. In some embodiments, the dimer-preventing nucleotide is the same nucleotide as the mutation-specific nucleotide.

While not wishing to be bound by any theory, we theorize that the mutation-specific nucleotide(s), complementary to the ultimate 3' base(s) of the mutation site of the target strand of the mutant DNA target sequence, favors the hybridization of the palm sequence with the mutant DNA target sequence at the palm target site of the mutant DNA target sequence. As the mutation-specific nucleotide of the mutation-recognizing sequence is mismatched to the mutation in the wild-type DNA target sequence, the palm target site of the wild-type DNA target sequence competes with the hairpin sequence for hybridization to the palm sequence significantly reducing the stable formation of a hybrid between the palm sequence and wild-type DNA target sequence.

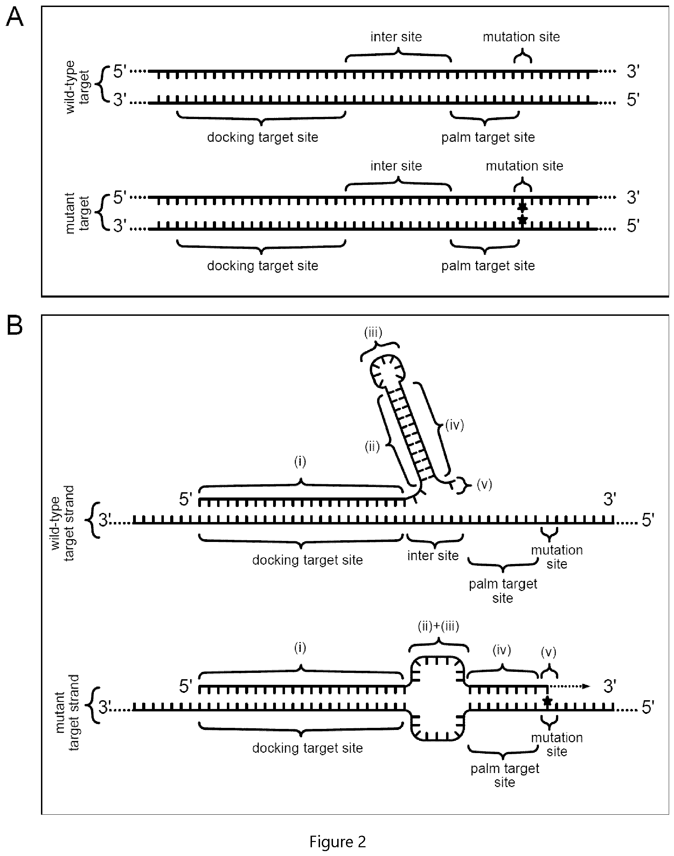

This is further illustrated in Figure 2 showing a possible interaction between an embodiment of a Switch primer according to the invention with its respective mutant and wild-type DNA target sequences, wherein the mutant DNA target sequence hybridizes with the palm sequence and the mutation-recognizing sequence of the Switch primer, while the wild-type DNA target sequence does not readily form such hybrids due to increased competition of hybrid formation between the hairpin sequence and the palm sequence of the Switch primer as a result of a mismatch of the mutation-specific nucleotide comprising the mutation-recognizing sequence.

In some embodiments the Switch primer is a nucleotide sequence according to SEQ ID NOs 9-22 and 32

### CHIMERIC SWITCH PRIMERS

The placement of RNA bases within conventional DNA primers has been shown to decrease primer dimer formation and increase the efficiency and specificity of the PCR reaction. Without wishing to be bound by any theory or mechanism of action, it seems that non-specific amplification products are generated mainly when base-pairing occurs between the 3' end of a primer and a complementary base on another primer or probe, or even when self-pairing occurs between the 3' end of a primer and a complementary base on the same primer.

It has been surprisingly found that the incorporation of at least one ribonucleotide within the Switch primer at distinctive positions can reduce or even prevent non-specific amplification products such as primer dimers further improving efficiency of mutant DNA target sequences amplification. Accordingly, in some embodiment, the multi-part amplification primer comprises at least one ribonucleotide. In a preferred embodiment, the at least one ribonucleotide is placed within the last 10 nucleotides of the 3' terminus of the primer, or up to 10 nucleotides upstream to the expected initiation of elongation position of the primer dimer, wherein no two ribonucleotides are adjacent to one another and the 3' terminal base is a deoxyribonucleotide.

### In some embodiments the Switch primer is a sequence according to SEQ ID NOs 33-35

### INDUSTRIAL APPLICATION

The present invention further relates to a method for primer-dependent amplification and detection, capable of amplifying and detecting at least one mutant DNA target sequence in a sample. Said method is capable of amplifying and detecting in a sample at least one mutant DNA target sequence in a mixture containing, for each mutant DNA target sequence, a closely related wild-type DNA target sequence that differs from the mutant DNA target sequence by as little as one or two base pairs utilizing the Switch primers according to the invention. Said method comprises steps (a) to (d).

In a first step (a) of the method according to the invention, a sample is provided comprising at least one mutant DNA target sequence, for which identification and/or detection is desired. Samples useful for the method according to the invention may be prepared according to standard procedures as known in the art such as nucleic acid purification, reverse transcription, concentration, or combinations thereof. For instance, samples may be prepared from biological/clinical samples of a subject such as, but not limited to, whole blood samples, blood plasma samples, or tissue samples. In a preferred embodiment, samples are prepared from liquid biopsies. In some embodiments provided samples comprise cfDNA isolated from blood plasma samples.

Further provided in step (a) is for each of the at least one mutant DNA target sequence a pair of a forward primer and a reverse primer, wherein the at least one of forward primer and reverse primer is a multi-part amplification primer according to the invention (Switch primer) characterized in that it is selective for the mutated DNA target sequence but mismatched to the wild-type DNAtarget sequence. In a preferred embodiment, said forward primer is a Switch primer.

In some embodiments, the reverse primer is a conventional primer. In some embodiments, the reverse primer is a multi-part amplification primer according to the invention. In the case that both forward and reverse primers are multi-part amplification primers, the forward primer has a mutation-specific nucleotide that is complementary to the mutation site in one of the two strands of the mutant DNA target sequence, and the reverse primer has a mutation-specific nucleotide that is complementary to the other mutant DNA target sequence of that base pair in the other of the two strands of the mutant DNA target sequence, whereby one primer binds to a target strand, and the other primer binds to the complementary target strand.

In a second step (b) a primer-dependent amplification mixture is prepared comprising or consisting of a DNA polymerase, deoxyribonucleoside triphosphates, an amplification buffer, other reagents required for amplification, the sample of step (a) and the pair of the forward primer and reverse primer for each mutant DNA target sequence of step (a).

In the following step (c), the mixture of step (b) is repeatedly cycled in a primer-dependent amplification reaction, under primer annealing conditions, having a primer-annealing temperature, to amplify each mutant DNA target sequence present in the sample, thereby generating amplification products of the mutant DNA target sequence. In some embodiments, the docking sequence of the multi-part amplification primer hybridizes at the primer annealing temperature of the docking sequence with the mutant DNA-target sequence and with its closely related wild-type DNA target sequence.

The use of amplification primers and primer-dependent amplification and detection of target sequences in general is well known by the skilled person in the art. Primer-dependent amplification reactions useful in methods according to this invention may be any suitable exponential amplification method, including, but not limited to, polymerase chain reaction (PCR), either symmetric or non-symmetric, ligase chain reaction (LCR), rolling circle amplification (RCA) or the likes. In a preferred embodiment, the method according to the invention utilizes PCR. Primer- dependent amplification reactions may comprise isothermal and/or repeated thermal cycles of primer annealing, primer extension, and strand denaturation.

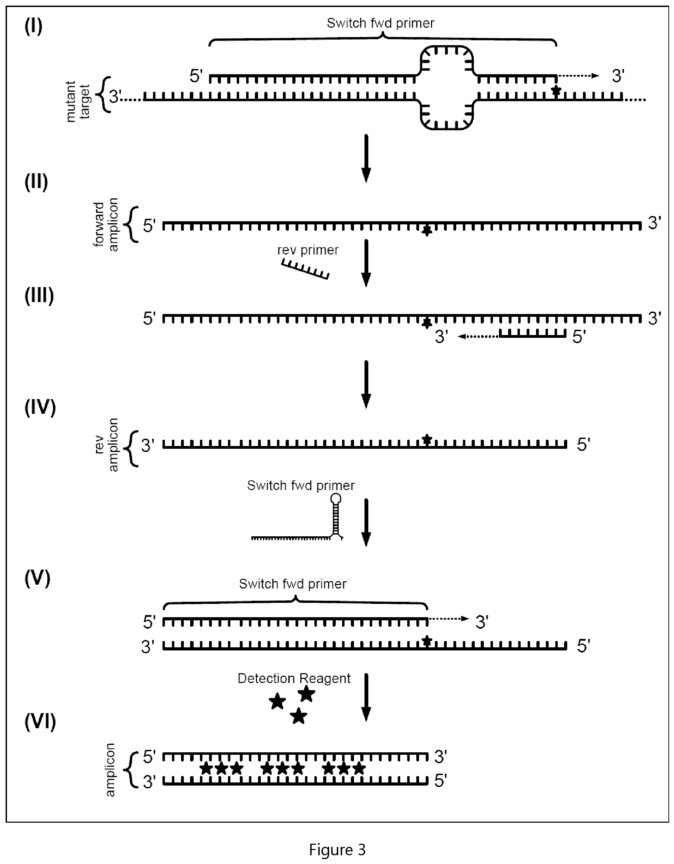

A primer-dependent amplification reaction is exemplarily depicted for illustration in Figure 3. First, a multi-part amplification forward primer (Switch fwd primer, Fig 3 I) anneals with the target strand of the mutant target sequence and a first extension product is generated (forward amplicon) by polymerase-dependent synthesis (Fig. 3 II). A second reverse primer (rev primer), such as a conventional primer, then binds the forward amplicon and initiates synthesis of a reverse amplicon (Fig 3 III). In a next step the forward primer hybridizes with the rev amplicon and initiates the synthesis of another forward amplicon.

In a last step (d), the at least one mutant DNA target sequence is detected by measuring the quantity of the amplification products obtained in step (c). Detection may be performed following amplification by methods known in the art such as gel electrophoresis. Alternately, homogeneous detection may be performed in a single tube, well, or other reaction vessel during (real time) or at the conclusion (end point) of the amplification reaction using reagents present during amplification. Detection reagents include double-stranded DNA binding dyes, for example SYBR Green, fluorescently labeled hybridization probes that allow signal detection upon hybridization such as molecular beacons, probes that are cleaved during amplification such as TaqMan probes, or any known detection reagent known by the skilled person in the art.

In some embodiments, said method allows the detection of at least one mutant DNA target sequence in a sample with as few as 1 copy of at least one mutant DNA target sequence in a mixture containing, for each mutant DNA target sequence, 100 copies of a closely related wild-type DNA target sequence that differs from the mutant DNA target sequence by as little as one or two base pairs. In a preferred embodiment, said method allows the detection of at least one mutant DNA target sequence in a sample with as few as 10 copies of at least one mutant DNA target sequence in a mixture containing, for each mutant DNA target sequence, 1,000 copies, more preferred 10,000 copies, of a closely related wild-type DNA target sequence that differs from the mutant DNA target sequence by as little as one or two base pairs

In diagnostic tests, the simultaneous amplification of multiple target sequences enables highly cost-effective and rapid testing of a range of biomarkers such as mutations within target sequences as well as genotyping. In some instances, the simultaneous amplification of multiple targets during for instance RT-PCR is necessary to measure target abundance in a single reaction. Accordingly, it is beneficial for such assays that Switch primers according to the invention efficiently amplifying their targets without interference from other primer pairs (background primers), such as conventional primers during the amplification and/or detection assay.

It has been surprisingly found that Switch primers according to the invention are able to selectively amplify mutant target DNA sequences according to the invention in the presence of multiple background primers. Accordingly, in some embodiments of the method according to the invention, the method is capable of amplifying and/or detecting in a sample at least one mutant DNA target sequence in a mixture containing, for each mutant DNA target sequence, a closely related wild-type DNA target sequence that differs from the mutant DNA target sequence by as little as one or two base pairs, wherein said sample further comprises at least one set of background primers, more preferably between about 1 to about 30, between about 1 to about 20, between about 1 to about 15, between about 5 to about 15, between about 5 to about 10, and most preferably between about 1 to about 20 background primers.

### SWITCH PRIMER DESIGN

The design of Switch primers according to the invention may require optimization depending on the selected target DNA sequence. As known in the art, primer design requires the careful selection of sequence and length that allow optimal amplification of the intended target sequence. For instance, adjusting the primer length in order to match amplification protocols and used machines for amplification and/or detection is critical for the successful amplification and faithful detection of the target sequence.

Switch primer design may require the adjustment of lengths and sequences of the five contiguous DNA sequences (i) to (v) comprising said primer according to the invention. For instance, shortening or increasing the length of the docking sequence to achieve a desired melting temperature may allow optimal binding of the docking sequence to the target DNA sequence, thus stabilizing further amplification of the mutant target DNA sequence. Additionally, adjusting the placement of the docking sequence by sliding a window of the desired length over the target DNA sequence may improve binding properties as known in the art. Similarly, adjustment of the hairpin structure, formed by the hairpin sequence, spacer sequence, and palm sequence, can impact optimal target engagement during amplification by allowing proper unfolding of the hairpin when engaging the respective mutant target DNA sequence comprising the mutation site.

While the skilled person in the art knows necessary steps to optimize primer sequences for conventional primers as well as for more sophisticated primer designs such as multi-part primers, it is suggested to first choose a Switch primer design with defined length of sequences (i) to (v) and placement of the mutation-specific-nucleotide at the first or second nucleotide of the mutation-recognizing sequence (v) for any desired target DNA sequence. Additionally, it is recommended to include a dimer-preventing nucleotide according to the invention and incorporate ribonucleotides within the Switch sequence at positions as specified in the description. It may be useful to optimize primers as specified above and test Switch sequences and amplification/detection protocols using a sample containing the mutated target DNA sequence and the wild-type DNA sequence at known concentrations using several trial runs varying multiple properties such as length and sequence of parts (i) to (v) of the Switch primer to ensure specificity and selectivity are sufficient for the detection of very low copy numbers of mutant target DNA sequences in a sample comprising wild-type target DNA sequences at high concentrations according to the invention. Several protocols and optimization procedures are shown in the EXAMPLE section. However, it should be noted that suggestions and examples for primer design optimization are purely for illustration and not limiting.

### KITS

Further disclosed are reagent kits for performing the method according to the invention.

Said kits can include one or more pairs of multi-part amplification primers according to the invention for one or more mutant DNA target sequence, dNTPs, a primer-dependent polymerase, a detection probe for each mutant DNA target and other reagents, particularly amplification buffer, needed for amplification. One primer of each primer pair can be a multi-part amplification primer, and the other primer of each pair can be a conventional primer or a multi-part amplification primer.

The kits can be used for any application which involves a nucleic acid amplification reaction, such as diagnostic kits for cancer screenings.

In some embodiments, the kit further comprises the detection reagent able to detect the mutant DNA target sequence.

### BRIEF DESCRIPTION OF THE FIGURES

**Figure 1** shows a schematic representation of an embodiment of a Switch primer according to this invention. Depicted are sequences (i) to (v) contiguous DNA sequences of the primer.
**Figure 2** panel A shows a depiction of a schematic representation of a mutant and wild-type DNA target sequence according to the invention. Panel B shows a schematic representation of a possible engagement of the Switch primer with either the mutant DNA target sequence or the wild-type DNA target sequence.
**Figure 3** shows a schematic representation of amplification cycles and detection of amplicons according to the method of the invention.

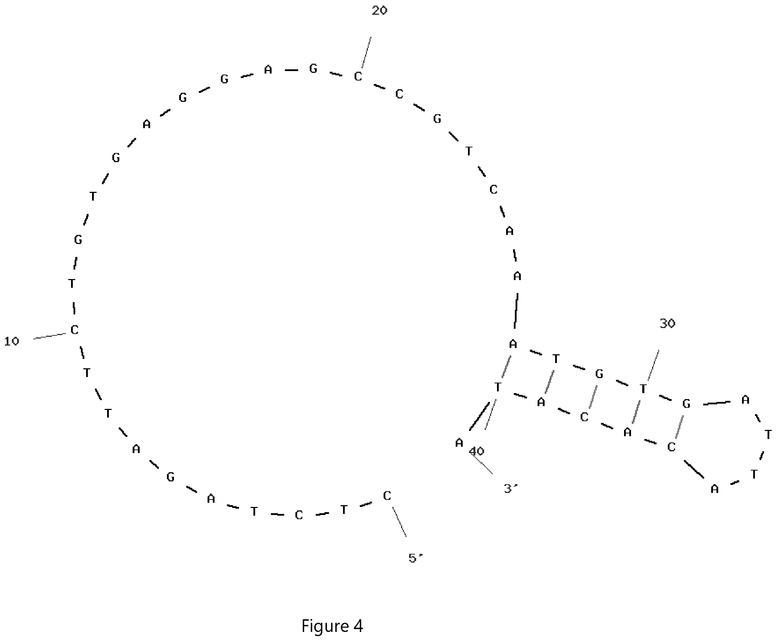

**Figure 4** shows the secondary structure of an exemplary embodiment of the Switch primer design according to the invention. Indicated are base, 5' and 3' ends, as well as base numbers counted from the 5' end. Bases 1 to 26 correspond to the docking sequence (i), bases 27 to 31 correspond to the hairpin sequence (ii), bases 32 to 35 to the spacer sequence (iii), bases 36 to 40 to the palm sequence (iv) and base 41 to the mutation-recognizing sequence (v) according to the invention.

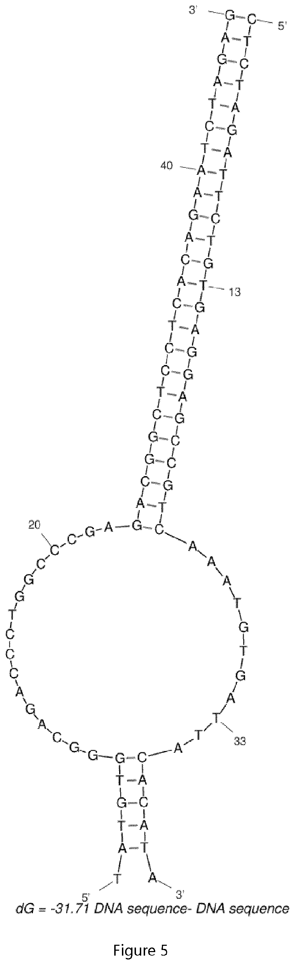

**Figure 5** depicts the interaction of the Switch primer of figure 4 (right strand) with its mutant DNA target sequence (left strand). Counted from the 5' first base of the mutant DNA target sequence, the first base correspond to the mutation site, bases 2 to 6 to the palm target site, bases 7 to 23 to the inter site, and bases 24 to 47 to the docking target site.

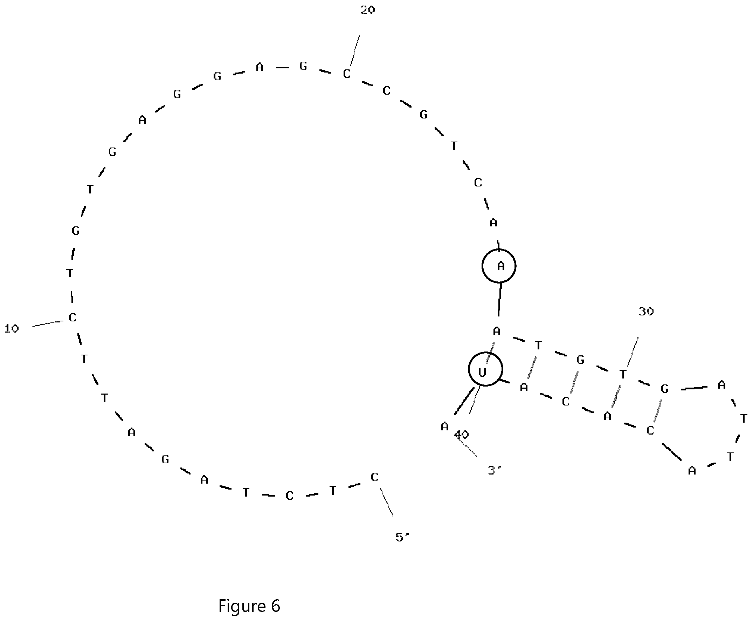

**Figure 6** depicts a particular embodiment of the invention in which the Switch primer of figures 4 and 5 comprises integrated ribonucleotides at position 26 and 40 (circled bases).
**Figure 7** shows the real-time qPCR results analysis of the PCR assay described in Example 1.
**Figure 8** shows the real-time qPCR results analysis of the PCR assay described in Example 2.
**Figure 9** shows the real-time qPCR results analysis of the PCR assay described in Example 3.
**Figure 10** shows the real-time qPCR results analysis of the PCR assay described in Example 4.
**Figure 11** shows the real-time qPCR results analysis of the PCR assay described in Example 5.

### EXAMPLES

### Example 1

### SWITCH PRIMER SELECTIVELY AMPLIFY MUATNT TARGET DNA SEQUENCES

In order to illustrate the increased selectivity and specificity of amplification of mutant DNA target sequences over wild-type DNA target sequences, real-time qPCR experiments were devised.

### DNA Templates

Three different DNA templates were utilized in the real-time PCR experiments. The first DNA template, designated as 'Mut gB', was a synthetic gBlock that contained a 250-base sequence comprising the mutation of interest (mutant DNA target sequences). The Mut gB were synthesized by IDT DNA Inc. and diluted to 4.15 nM and further diluted for different experiments as indicated. The second DNA template, designated as 'WT', was extracted from Glioblastoma U87 cell line and comprised the wild-type DNA target sequence. The genomic DNA was diluted to a 340 ng/µl stock solution and was further diluted as indicated. Wild-type and mutant DNA target sequence differed in a single mutation (mutation site). A third DNA template was a mixture of Mut gB and WT DNA at varying ratios, allowing the assessment of the PCR assay's sensitivity and specificity for detecting the mutation of interest. Positive control primers, designated as 'Region' primers, were used to determine the copy-number ratio between the Mut gB and WT DNA templates. The 'Region' primers targeted the sequence adjacent to the tested mutation and served as a positive control and quantitative normalization for the PCR assay.

### Primers and Probes

Switch primers targeting the following mutation of indicated human genes (mutant target DNA sequences) were designed: KRAS G12D (SEQ ID NO 1), EGFR L858R (SEQ ID NO 3), CTNNB1 S33C (SEQ ID NO 2) and SMAD4 R361H (SEQ ID NO 4). Wild-type DNA sequences differed from mutant DNA sequences in a single mutation (SNP), wherein the wild-type target DNA sequence is for KRAS target SEQ ID NO 5, EGFR target SEQ ID NO 6, CTNNB1 target SEQ ID NO 7, and for SMAD4 target SEQ ID NO 8. Two types of experiments were performed: single-and multiplexed experiments. In the singleplex experiments, each reaction tube consisted of a single primer set. In the multiplex experiments, each reaction tube consisted of the tested primer set and a set of additional 20 primer sets targeting different cancer mutations serving as background. For the singleplex primer mix, the primers were prepared by diluting them to a 4 mM stock solution and further diluted to a final concentration of 200 nM in the reaction tube. Each reaction contained a primer set consisting of a forward Switch primer and a standard DNA reverse primer at equal concentrations. For the multiplex primer mix, each primer was diluted to 100 uM stock solution. The forward and reverse primers were then mixed to two separate primer mixes, target and background, each at a final concentration of 4 uM primer mix and 200 nM in the reaction tube.

The multiplex experiments were performed using TaqMan Probes dual labeled with 5' Fam and Black Hole Quencher 1. Probes were diluted with the target primer set to a final concentration of 3 uM in the target primer mix and 150 nM in the reaction tube.

In addition to regular Switch DNA primers, Switch Chimera (designated Switch-Chi) primers targeting SMAD4 R361H were also designed. In the Switch Chimera primers, two positions were modified with an RNA base. The positions of the RNA chimeric bases were chosen to be in the putative DNA polymerase initiation of the elongation site. Specifically, the first site was placed near the suspected position for self-elongation, and the second site was located nearest to the mutation site at the 3' end.

All primers and probes were synthesized by IDT DNA Inc.

### Real-Time PCR Setup

The Real-Time PCR enzyme mixture utilized either the ORA^{™} SEE qPCR Green ROX L Mix (HighQu GmbH, Germany) for SyBR green reactions or, the ORA^{™} SEE qPCR Probe Mix (HighQu GmbH, Germany) for TaqMan probe reactions.

Syber green Real-Time reaction protocol:

| | |
|---|---|
| 10 ul | ORA SEE qPCR mix |
| 1 ul | Primer set (4 mM) |
| 4 ul | Ultra-pure water |
| 5 ul | DNA template |

Taqman Real-Time reaction protocol:

| | |
|---|---|
| 10 ul | ORA SEE qPCR mix |
| 1 ul | Target primer (4 mM) and probe (3 mM) mix |
| 2 ul | Background primer mix (4 mM) |
| 2 ul | Ultra-pure water |
| 5 ul | DNA template |

All the Real-Time PCR experiments were performed on the Opus CFX 96 (Bio-Rad, USA)., with the following thermocycler program as depicted in Table 1.

**Table 1: RT-PCR cycle program**

| Step | Temperature (°C) | Time (minutes: seconds) |
|---|---|---|
| 1 | 95.0 | 3:00 |
| 2 | 95.0 | 0:10 |
| 3 | 67.0 | 0:10 |
| | Decrement temperature by -0.5 °C per cycle | |
| 4 | 72.0 | 0:30 |
| 5 | GOTO 2, 9 more times | |
| 6 | 95.0 | 0:10 |
| 7 | 62.0 | 0:10 |
| 8 | 72.0 | 0:30 |
| | + Plate Read | |
| 9 | GOTO 6, 49 more times | |
| 10 | Melt Curve 65.0 to 95.0 C, increment 0.5 C, 0:05 + Plate Read | |
| 11 | 4.0 | 1:00 |
| | END | |

In the TaqMan protocol the Melting Curve Protocol (stage number 10 in the program) was omitted, and the fluorophore replaced from SYBR green to FAM or HEX.

### Real-Time PCR data analysis

Sensitivity values were calculated using the following formula: Cq [NTC] - Cq [target], where NTC is the negative template control.

Specificity values were calculated using the following formula: Cq [WT] - Cq [mut].

In cases where no Cq values were obtained due to no amplification, a constant value of Cq=38 was assigned. This value was determined by calculating the average value of the normally distributed Cq values from all the negative template control (NTC) reactions that were previously performed.

### Results

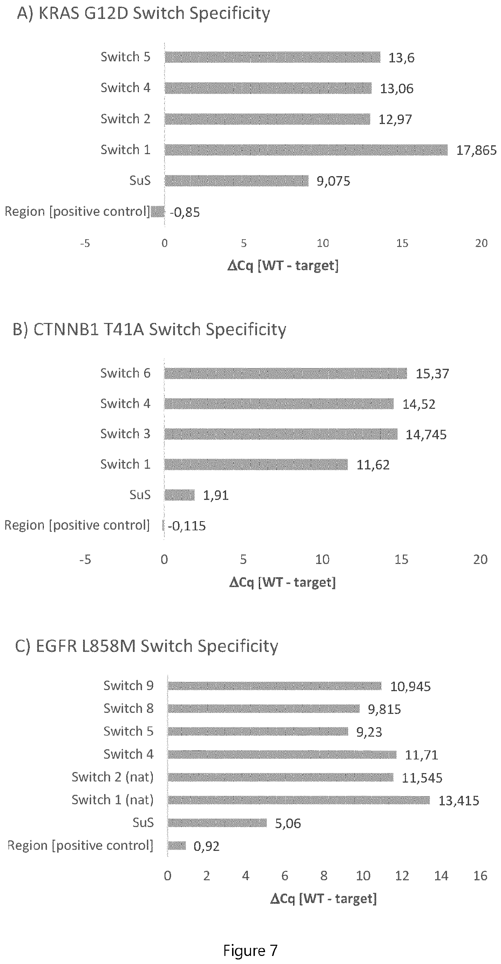

Initially, a set of Switch primers were designed for several cancer mutations to determine the best-performing design. Switch forward primers specifically targeting mutant target DNA sequences of KRAS were KRAS_Switch 1, 2, 4, 5 (SEQ ID NOs 9, 10, 11, 12 respectively), for CTNNB1 CTNNB1_ Switch 1, 3, 4, 6 (SEQ ID NOs 13, 14, 15, 16 respectively), and for EGFR EGFR_Switch 1 nat, 2 nat, 4, 5, 8, 9 (SEQ ID NOs 17, 18, 19, 20, 21, 22 respectively). The reverse primers of each primer set used were the same for all region-specific sets. For KRAS-specific amplification (Figure 7 A), KRAS_rev primers (SEQ ID NO 23), for CTNNB1-specific amplification (Figure 7 B) CTNNB1_rev primers (SEQ ID NO 24), and for EGFR-specific amplification (Figure 7 C) EGFR_rev primers (SEQ ID NO 25), were used. A positive control was included, wherein the forward primer was a conventional primer, which does not discriminate between the mutant and wild-type target DNA sequence (region, positive control). For KRAS-specific amplification (Figure 7 A), KRAS_fwd primers (SEQ ID NO 26), for CTNNB1-specific amplification (Figure 7 B) CTNNB1_fwd primers (SEQ ID NO 27), and for EGFR-specific amplification (Figure 7 C) EGFR_fwd primers (SEQ ID NO 28), were used. A reference reaction was performed using state-of-the-art primer designs enabling discrimination between mutant wildtype sequences (SuS-primer strategy, Kramer FR, Vargas DY. SuperSelective primer pairs for sensitive detection of rare somatic mutations. Sci Rep. 2021 Nov 17;11 (1):22384. doi: 10.1038/s41598-021-00920-4. PMID: 34789731; PMCID: PMC8599793.) SuS forward primers for KRAS-specific amplification (Figure 7 A), KRAS_SuS primers (SEQ ID NO 29), for CTNNB1-specific amplification (Figure 7 B) CTNNB1_SuS primers (SEQ ID NO 30), and for EGFR-specific amplification (Figure 7 C) EGFR_SuS primers (SEQ ID NO 31), were used. The charts depicted in Figure 7 A-C present the specificity of Switch primers in singleplex experiments targeting KRAS G12D, CTNNB1 S33C and EGFR L858R (mutant DNA target sequences) in a sample comprising both mutant and wildtype target DNA sequences. For each mutation, most of the Switch primers exhibited superior performance compared to the previously published Super Selective (Sus) primers. Several primers exhibited 1000-fold higher specificity to the mutant sequence compared to the wild-type (ΔCq 9-11) and others exhibited more than a 10,000-fold increase in specificity to the mutation (ΔCq > 13).

### Example 2

### HIGHLY SPECIFIC AMPLIFICATION OF MUTANT TARGET DNA SEQUENCES

The identification of mutant target sequences in samples containing low copies of the mutant and high copy numbers of the wild-type DNA target sequence is critical for the identification of ctDNA. In order to demonstrate the ability of the Switch primers to specifically identify low copy numbers of mutant DNA target sequences in the abundant presence of the corresponding wild-type DNA target sequence, multiple dilutions of mutant and wild-type sequence were prepared. The RT-PCR assay was performed and analyzed as described in Example 1.

In detail, the sensitivity of the Switch primer set 1 targeting KRAS G12D mutation was examined by combining mutant and wild-type templates at increasing serial dilutions, going up to 1:1000 mutant to wild-type ratio.

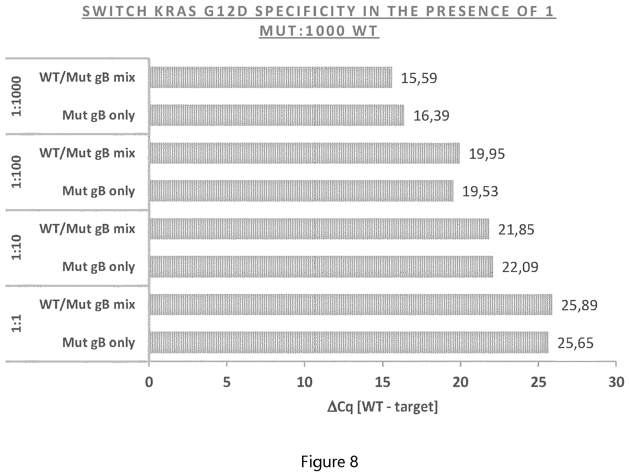

The results are summarized in Figure 8. There was no significant effect of the addition of wild-type genomic DNA to the specificity of KRAS Switch 1 to the KRAS G12D mutation. The specificity varies from ΔCq of 15.59 (above 10,000-fold) in 1:1000 ration of the mutated gBlock to 25.65 in 1:1 ratio.

### Example 3

### SWITCH PRIMERS EFFICIENTLY AMPLIFY TARGET SEQUENCES IN THE PRESENCE OF BACKGROUND PRIMERS

In some instances, the simultaneous amplification of multiple targets during RT-PCR is necessary to measure target abundance in a single reaction. Accordingly, it is beneficial for such assays that primers are efficiently amplifying their targets without interference from other primer pairs (background primers). The RT-PCR assay was performed and analyzed as described in Example 1.

Accordingly, for the next experiment, a multiplex assay was performed, in which the performance of switch primer set 1 targeting KRAS G12D was examined in the presence of 20 background primers with increasing serial dilution of mutant/ wild-type templates.

The Switch primers presented high specificity in a presence of 1:100 mutation to wild-type molecules in the presence of 20 background primer sets in the tube.

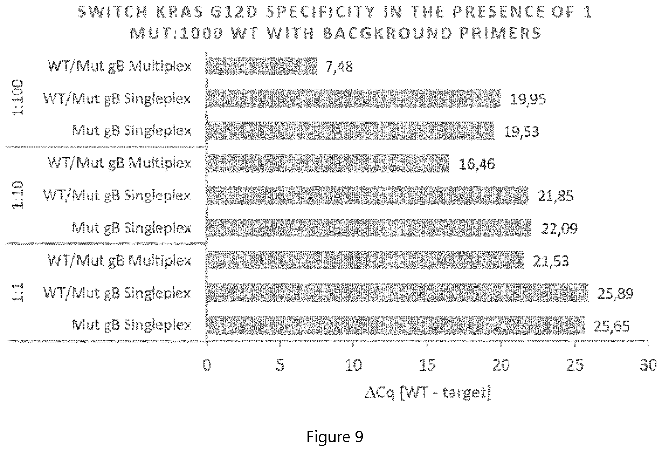

The results are depicted in Figure 9.

### Example 4

### CHIMERIC SWITCH PRIMERS

In the following experiment, 1-2 base positions of the Switch forward primer (SMAD4_Switch 3, SEQ ID NO 32) specifically amplifying SAMD4 mutant target DNA sequences were modified from DNA to RNA bases to create a Switch Chimeric primer. The first modified position was at the nearest neighbor to the 3' end base of the primer (SMAD4_Switch chi 3.1, SEQ ID NO 33). The second modified position was at the middle of the primer, near the predicted self-elongation site of the primer (SMAD4_Switch chi 3.2, SEQ ID NO 34). In addition, a third primer was designed, in which both sites were modified (SMAD4_Switch chi 3.3, SEQ ID NO 35). The Switch primers target the SMAD4 R361H mutation and were tested in a singleplex setting. As positive control a conventional forward primer was used amplifying both mutant and wild type target DNA sequence (SMAD4_region_fwd, SEQ ID NO 36). The reverse primer for all reaction was SMAD4_rev (SEQ ID NO 37). The RT-PCR assay was performed and analyzed as described in Example 1.

DNA to RNA modification at the nearest neighbor to the 3' end base of the primer (chi 3.1) increases the specificity by 3.5-fold (1.84 cycles). RNA modification at the middle of the primer, near the predicted self-elongation site of primer (chi 3. 2), increases the specificity by 36-fold (5.16 cycles). RNA modification on both positions (chi 3.3) increases the specificity by 150-fold (7.29 cycles).

The results show that modifying both positions of the Switch primer has improved its specificity compared to one RNA modification.

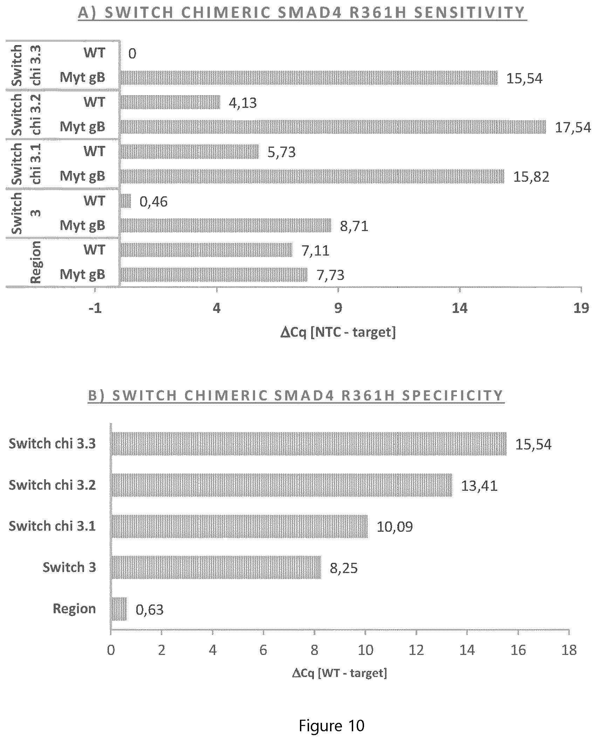

The results are presented in Figure 10. As shown, the incorporation of ribonucleotides within the Switch primer significantly increases specificity.

### Example 5

### POSITIONOING OF THE MUTATION-SPECIFIC NUCLEOTIDE

In some embodiments of the invention, the mutation-specific nucleotide comprising the mutation recognizing sequence of the Switch primer, may be placed at the second position of the sequence. In order to demonstrate the impact of positing of interrogating base on specificity, two Switch designs were investigated. Switch primer C420R switch 2 (SEQ ID NO 38) comprises the mutation-specific nucleotide at the first position, while Switch primer 3' 2nd position (SEQ ID NO 39) comprises the mutation-specific nucleotide at the second position of the mutation-recognizing sequence. Both sequences were designed for a PIK3CA specific mutation (PI3KCA mutant DNA target sequence SEQ ID NO 40, PI3KCA wild-type DNA target sequence SEQ ID NO 41). The reverse primer for all reactions was SEQ ID NO 42, while the positive control (region control, conventional fwd primer) was SEQ ID NO 43. The RT-PCR assay was performed and analyzed as described in Example 1.

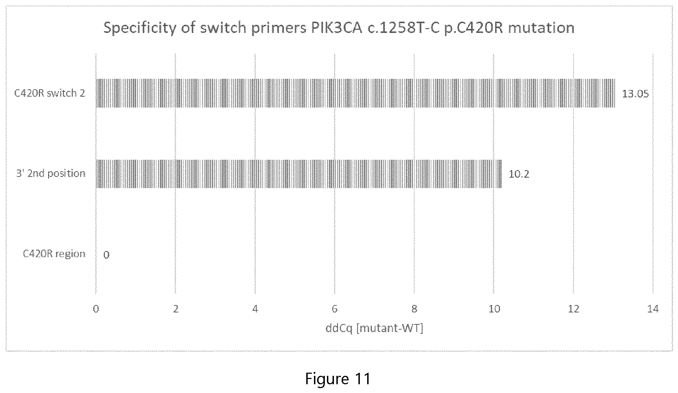

Switch 2, which targets the PIK3CA C420R mutation, demonstrated high specificity with ΔΔCq value of 13.05, 1/10000 mut/w.t. The primers with the mutation complementary base at the second position upstream to the 3' end demonstrate ΔΔCq value 10.2. This ratio is more than 1/1000 mut/w.t. This ratio is sufficient for ctDNA samples. This result shows that the position of the mutation complementary base can also be at the second position from the 3' end of the primer. The results are depicted in Figure 11.

## Claims

1. A multi-part amplification primer that distinguishes between a mutant DNA target sequence and a closely related wild-type DNA target sequence **characterized in that** it consists of or comprises, in the 5' to 3' direction, the following five contiguous DNA sequences,
(i) a docking sequence, capable of forming a hybrid 15-40 nucleotides in length with the mutant DNA target sequence and with the wildtype DNA target sequence,
(ii) a hairpin sequence incapable of forming a hybrid with the mutant DNA target sequence and with the wildtype DNA target sequence,
(iii) a spacer sequence,
(iv) a palm sequence complementary to the mutant DNA target sequence and wildtype DNA target sequence capable of forming a hybrid 4 to 10 nucleotides in length with the hairpin sequence and
(v) a mutation-recognizing sequence, comprising a mutation-specific nucleotide at the first or second 5'position of the sequence,
wherein
the contiguous hairpin sequence, spacer sequence, and palm sequences form a stem-loop structure,
the mutant target DNA sequence is perfectly complementary to the contiguous sequence formed by the palm sequence and the mutation-recognizing sequence, and the wild-type DNA target sequence is mismatched to said contiguous sequence with the mutation-specific nucleotide of the mutation-recognizing sequence.

2. The multi-part amplification primer of claim 1, wherein the docking sequence further comprises at the ultimate 3'position a dimer-preventing nucleotide that does not pair with the mutation-specific nucleotide and/or with any other nucleotide at the 3' end position of the switch primer.

3. The multi-part amplification primer of claim 2, wherein said dimer-preventing nucleotide is the same nucleotide as the first 5' nucleotide of the mutation-recognizing sequence.

4. The multi-part amplification primer of claim 2 or 3, wherein said dimer-preventing nucleotide is a locked nucleic acid (LNA).

5. The multi-part amplification primer of any of claims 1 to 4, further comprising at least one ribonucleotide.

6. The multi-part amplification primer of claim 5, wherein said the at least one ribonucleotide is placed within the last 10 nucleotides of the 3' terminus of the primer, no two ribonucleotides are adjacent to one another, and the 3' terminal base is a deoxyribonucleotide.

7. A primer-dependent amplification and detection method that is capable of amplifying and detecting in a sample at least one mutant DNA target sequence in a mixture containing, for each mutant DNA target sequence, a closely related wild-type DNA target sequence that differs from the mutant DNA target sequence by as little as one or two base pairs, comprising consisting or essentially consisting of the following steps:
(a) providing a sample comprising the at least one mutant and/or wild-type DNA target sequence, and for each mutant DNA target sequence a pair of a forward primer and a reverse primer,
(b) preparing a primer-dependent amplification reaction mixture comprising or consisting of a DNA polymerase, deoxyribonucleoside triphosphates, an amplification buffer, other reagents required for amplification, the sample and the pair of the forward primer and reverse primer for each mutant DNA target sequence of step (a),
(c) repeatedly cycling the mixture thus obtained in a primer-dependent amplification reaction, under primer annealing conditions, having a primer-annealing temperature, to amplify each mutant DNA target sequence present in the sample, and
(d) detecting said at least one mutant DNA target sequence by measuring the quantity of the amplification products thus obtained;
wherein
the forward and/or reverse primer of the primer pair for each mutant DNA target sequence is a multi-part amplification primer according to any of the preceding claims **characterized in that** it is specific for the mutated DNA target sequence but mismatched to the wild-type DNA target sequence,
the docking sequence hybridizes at the primer-annealing temperature with the mutant DNA target sequence and with its closely related wild-type DNA target sequence.

8. The method of claim 7, wherein either the forward or reverse primer of the primer pair for each mutant DNA target sequence is a conventional primer.

9. The method of claim 7 or 8, wherein if the docking sequence and the palm sequence of the primer are hybridized either to the mutant DNA target sequence or to the wild-type DNA target sequence,
and wherein the probability that during said cycling a multi-part amplification primer/wild-type DNA target sequence hybrid will be extended is at least 1,000 times lower than the probability that during said cycling a multi-part amplification primer/mutant DNA target sequence hybrid will be extended as evidenced by a difference in cycle numbers (ΔCq) of at least ten thermal cycles.

10. The method according to any of claims 7 to 9, wherein the forward and reverse primers of said pair of forward primer and reverse primer are multi-part amplification primers according to any of claims 1 to 6,
the forward primer has an mutation-specific nucleotide that is complementary to the mutation site in one of the two strands of the mutant DNA target sequence,
the reverse primer has an mutation-specific nucleotide that is complementary to the other mutant DNA target sequence of that base pair in the other of the two strands of the mutant DNA target sequence, whereby one primer binds to a target strand,
and the other primer binds to the complementary target strand.

11. The method according to any of claims 7 to 10, wherein the closely related wild-type DNA target sequence differs from the mutant DNA target sequence by one base.

12. The method of any of claims 7 to 11, wherein the docking sequence hybridizes with the mutant and wild-type DNA target sequence at the primer annealing temperature.

13. The method of any of claims 7 to 12, wherein the primer-dependent amplification mixture of step (b) further comprises homogeneous fluorescence detection means for detecting amplification products, and
step (d) comprises detecting said at least one mutant DNA target sequence by measuring the intensity of fluorescence from said homogeneous fluorescence detection means.

14. The method of claim 13, wherein the primer-dependent amplification and detection method is a polymerase chain reaction (PCR), and detection is real-time (RT) detection.

15. The method of any of claims 7 to 14, wherein method is capable of amplifying and detecting in a sample as few as 10 copies of at least one mutant DNA target sequence in a mixture containing, for each mutant DNA target sequence, 10,000 copies of a closely related wild-type DNA target sequence that differs from the mutant DNA target sequence by as little as one or two base pairs.
